Europäisches Patentamt

**European Patent Office**

**Office européen des brevets**

(19)

(11) Publication number: **0 175 498**
A2

# (12) EUROPEAN PATENT APPLICATION

(21) Application number: **85305994.7**

(22) Date of filing: **22.08.85**

(51) Int. Cl.⁴: **C 07 K 5/06**, C 07 K 5/08, A 61 K 37/02

(30) Priority: **23.08.84 US 643655**

(43) Date of publication of application: **26.03.86** Bulletin 86/13

(84) Designated Contracting States: **AT BE CH DE FR GB IT LI LU NL SE**

(71) Applicant: **ADMINISTRATORS OF THE TULANE EDUCATIONAL FUND, 218 Gibson Hall Tulane University, New Orleans Louisiana (US)**

(72) Inventor: **Coy, David H., 4319 Perrier Street, New Orleans Louisiana 70115 (US)**

(74) Representative: **Deans, Michael John Percy et al, Lloyd Wise, Tregear & CO. Norman House 105-109 Strand, London WC2R OAE (GB)**

(54) **Peptides, their use in medicine and methods for their preparation.**

(57) A tripeptide has the formula

A-B-C

wherein A is pGlu, D-pGlu, or N-AcSar; B is Phe, D-Phe, Tyr, β-Nal, D-β-Nal, p-Cl-Phe, or D-p-Phe; and C is Gly or NHR, wherein R is a lower alkyl group; or a pharmaceutically acceptable salt thereof, provided that, when A is pGlu, B cannot be Phe. Medical use. e.g. in the treatment of gastric hypersecretion, is disclosed.

To prepare the tripeptide, a resin of C and a blocking group, and a resin of B and a blocking group are first prepared. The C-containing resin and the B-containing resin are reacted to form a reaction product. The reaction product is reacted with an A-containing resin to form a further reaction product which is treated to yield the tripeptide A-B-C.

ACTORUM AG

Peptides, their use in Medicine and
Methods for their Preparation

This invention relates to peptides, their use in medicine, and methods for their preparation.

The reader is referred to Schally et al, U.S. Pat. No. 4,328,134, the disclosure of which is to be regarded as incorporated herein by reference for a description of tripeptides said to exhibit anorexigenic properties and to inhibit gastric hypersecretion.

The invention features, in one aspect thereof, a tripeptide having the formula

A-B-C

wherein A is pGlu, D-pGlu, or N-AcSar; B is Phe, D-Phe, Tyr, β-Nal, D-β-Nal, p-Cl-Phe, or D-p-Cl-Phe; and C is Gly or NHR, wherein R is a lower (6 or fewer carbon atoms) alkyl group; or a pharmaceutically acceptable salt thereof, provided that, when A is pGlu, B cannot be Phe. (When neither the D- or L-designation is given, the L-isomer is intended.)

In preferred embodiments, the tripeptide is pGlu-D-Phe-Gly; N-AcSar-Phe-Gly; pGlu-Tyr-Gly; pGlu-β-Nal-Gly; pGlu-p-Cl-Phe-Gly; pGlu-D-β-Nal-Gly; or pGlu-D-p-Cl-Phe-Gly; or a pharmaceutically acceptable salt thereof.

In other preferred embodiments, a therapeutically effective amount of the therapeutic compound and a pharmaceutically acceptable carrier substance, e.g., magnesium carbonate or lactose, form a therapeutic composition, e.g., a pill, tablet, capsule, or liquid for oral administration to a patient; a liquid capable of being administered nasally to a patient; or a liquid capable of being administered intravenously, parenterally, subcutaneously, or intraperitoneally.

We have found examples of tripeptides in accordance with the invention to be effective in inhibiting gastric hypersecretion and associated physiological disorders, e.g., gastric, peptic, or duodenal ulcers. They also exhibit a long duration of activity, thus requiring lower effective dosages.

The invention provides, in a second and alternative aspect thereof, a method of making a tripeptide having the formula

A-B-C

wherein A is pGlu, D-pGlu, or N-AcSar; B is Phe, D-Phe, Tyr, β-Nal, D-β-Nal, p-Cl-Phe, or D-p-Cl-Phe; and C is Gly or NHR, wherein R is a lower alkyl group, provided that, when A is pGlu, B cannot be Phe; said method comprising:

(a) preparing a resin of C and a blocking group;

(b) preparing a resin of B and a blocking group;

(c) reacting said C-containing resin and said B-containing to form a reaction product;

(d) reacting an A-containing resin with said reaction product to form a further reaction product; and

(e) treating said further reaction product to yield a tripeptide of formula A-B-C.

We now describe the structure, synthesis, and use of preferred embodiments of the invention.

Structure

The tripeptides have the general formula A-B-C defined above. They can also be provided in the form of pharmaceutically acceptable salts. Examples of preferred salts are those with therapeutically acceptable organic acids, e.g., acetic, lactic, maleic, citric, malic, ascorbic, succinic, benzoic, salicyclic, methanesulphonic, toluenesulphonic, or pamoic acid, as well as polymeric acids such as tannic acid or carboxymethyl cellulose, and salts with inorganic acids such as the hydrohalic acids, e.g, hydrochloric acid, sulphuric acid, or phosphoric acid.

Synthesis

The synthesis of specific tripeptides follows by way of example. Other tripeptides within the scope of this invention can be prepared by making appropriate modifications of the following synthetic method.

pGlu-D-Phe-Gly

The first step is the preparation of pyroglutamic acid-D-phenylalanine-glycine-resin, as follows.

Alpha-t-butoxycarbonyl (Boc)-glycine is esterified to commercially available chloromethylated polystyrene resin (1% cross-linked with divinylbenzene) to give a Boc-Gly-resin with an incorporation of 0.72 mmole of Gly per gram. This resin (1.38g, 1.0 mmole) is placed in the reaction vessel of a Beckman 990B peptide synthesizer programmed to perform the following reaction cycle: (a) $CH_2Cl_2$: (b) 33% trifluoroacetic acid in $CH_2Cl_2$ (2 times for 1 and 25 min each); (c) $CH_2Cl_2$: (d) ethanol; (e) $CH_2Cl_2$: (f) 10% triethylamine in $CHCl_3$; (g) $CH_2Cl_2$.

The neutralized resin is stirred with Boc-D-phenylalanine and diisopropylcarbodiimide (3.0 mmole) in $CH_2Cl_2$ for 1 hour, and the resulting amino acid resin is then cycled through steps (a) to (g) in the above wash program. Pyroglutamic acid (3.0 mmole) is then coupled by the same procedure. After washing and drying, the completed resin weighs 1.46g.

The completed pyroglutamic acid-D-phenylalanine-glycine resin is mixed with anisole (4ml), dithiothreitol (100mg), and anhydrous hydrogen fluoride (36ml) at 0°C and stirred for 45 minutes. Excess hydrogen fluoride is evaporated rapidly under a stream of dry nitrogen and free peptide precipitated and washed with ether. The crude peptide is then dissolved in a minimum volume of 2M acetic acid and eluted on a column (2.5 X 100mm) of Sephadex G-25 in the same solvent. Fractions containing a major component, as determined by uv absorption and thin layer chromatography (tlc), are then pooled, evaporated to a small volume and applied to a column (1.5 X 150cm) of silica gel, which is eluted with 1-butanol:acetic acid:water:ethyl acetate (1:1:1:1 by volume).

Fractions are examined by tlc and analytical high performance liquid chromatography (hplc), and

- 4 -

pooled to give maximum purity. Repeated lyophilization of the solution from dilute acetic acid gives 145mg of the product as a fluffy white powder. This material is found to be homogeneous by hplc and tlc. Amino acid analysis of an acid hydrolysate confirms the composition of the tripeptide.

N-AcSar-Phe-Gly, pGlu-Tyr-Gly, pGlu-$\beta$-Nal-Gly, pGlu-p-Cl-Phe-Gly, pGlu-D-$\beta$-Nal-Gly, and pGlu-D-p-Cl-Phe-Gly are prepared in similar yields in an analogous fashion by appropriately modifying the above procedure. For example, to make N-AcSar-Phe-Gly, the resin is prepared as above, substituting Boc-sarcosine for the last amino acid, followed by removal of the Boc group and subsequent acetylation of the $\alpha$-amino group with 5% acetic anhydride. The product is prepared via hydrogen fluoride treatment of the resulting resin.

Use

When administered to a patient (e.g. orally, intravenously, parentally, nasally, or by suppository), the tripeptides are effective in inhibiting gastric hypersecretion, and can be used in the treatment of gastric, peptic, and duodenal ulcers, Zollinger-Ellison syndrome (gastrinoma), short bowel syndrome, reflex esophagitis, acute erosive gastritis, and pancreatic insufficiency.

The tripeptides can be administered to a patient in a dosage of 1mcg/kg/day to 250mcg/kg/day, preferably 5 to 100mcg/kg/day.

CLAIMS

1.      A tripeptide having the formula
                    A-B-C
wherein A is pGlu, D-pGlu, or N-AcSar; B is Phe, D-Phe,
Tyr, β -Nal, D- β -Nal, p-Cl-Phe, or D-p-Cl-Phe; and C
is Gly or NHR, wherein R is a lower alkyl group; or a
pharmaceutically acceptable salt thereof, provided that,
when A is pGlu, B cannot be Phe.

2.      A tripeptide according to Claim 1, having the
formula pGlu-D-Phe-Gly, N-AcSar-Phe-Gly, pGlu-Tyr-Gly,
pGlu- β -Nal-Gly, pGlu-p-Cl-Phe-Gly, pGlu-D- β -Nal-Gly,
pGlu-D-p-Cl-Phe-Gly; or a pharmaceutically acceptable salt
thereof.

3.      A tripeptide according to Claim 1 or Claim 2 for
use in medicine.

4.      A tripeptide according to Claim 1 or Claim 2 for
use in the treatment of gastric hypersecretion.

5.      A therapeutic composition comprising a
therapeutically effective amount of a compound according
to any preceding Claim, together with a pharmaceutically
acceptable carrier substance.

6.      A composition according to Claim 5, wherein said
composition is in the form of a pill, tablet, capsule, or
liquid for oral administration to a patient, or in the form
of a liquid capable of being administered nasally as drops
or spray to a patient, or in the form of a liquid capable
of being administered intravenously, subcutaneously,
parenterally, or intraperitoneally to a patient.

7.      A method of making a tripeptide having the formula
                    A-B-C
wherein A is pGlu, D-pGlu, or N-AcSar; B is Phe, D-Phe,
Tyr, β -Nal, D- β -Nal, p-Cl-Phe, or D-p-Cl-Phe; and C
is Gly or NHR, wherein R is a lower alkyl group, provided
that, when A is pGlu, B cannot be Phe; said method
comprising:

(a)    preparing a resin of C and a blocking group;

(b)    preparing a resin of B and a blocking group;

(c)    reacting said C-containing resin and said B-containing to form a reaction product;

(d)    reacting an A-containing resin with said reaction product to form a further reaction product; and

(e)    treating said further reaction product to yield a tripeptide of formula A-B-C.